# NEUE EUROPÄISCHE PATENTSCHRIFT

(11) **EP 2 635 592 B2**
(45) Veröffentlichungstag und Bekanntmachung des Hinweises auf die Entscheidung über den Einspruch: **21.05.2025**
(45) Hinweis auf die Patenterteilung: 16.08.2017
(21) Anmeldenummer: 11784965.3
(22) Anmeldetag: 02.11.2011
(51) Int. Cl.: C07D 311/58, C11C 3/00, C07D 311/72, C07J 9/00

(54) **VERFAHREN ZUR GEWINNUNG VON PHYTOSTEROLEN UND/ODER TOCOPHEROLEN AUS RÜCKSTÄNDEN EINER DESTILLATION VON ESTERN PFLANZLICHER ÖLE, VORZUGSWEISE AUS DESTILLATIONSRÜCKSTÄNDEN AUS EINER UMESTERUNG VON PFLANZLICHEN ÖLEN**
METHOD FOR OBTAINING PHYTOSTEROLS AND/OR TOCOPHEROLS FROM RESIDUE OF A DISTILLATION OF THE ESTERS OF VEGETABLE OILS, PREFERABLY FROM DISTILLATION RESIDUE FROM A TRANSESTERIFICATION OF VEGETABLE OILS
PROCÉDÉ D'EXTRACTION DE PHYTOSTÉROLS ET/OU DE TOCOPHÉROLS À PARTIR DE RÉSIDUS D'UNE DISTILLATION D'ESTERS D'HUILES VÉGÉTALES, DE PRÉFÉRENCE À PARTIR DE RÉSIDUS DE DISTILLATION ISSUS D'UNE TRANSESTÉRIFICATION D'HUILES VÉGÉTALES

(30) Priorität: 03.11.2010 DE 102010050293
(43) Veröffentlichungstag der Anmeldung: 11.09.2013
(73) Patentinhaber: Verbio SE, 06780 Zörbig (DE)
(72) Erfinder: LEMP, Joachim, 04509 Delitzsch (DE); BAADE, Nico, 06449 Aschersleben / OT Schackstedt (DE); PÖHLS, Emanuel, 04155 Leipzig (DE)
(74) Vertreter: Wallinger Ricker Schlotter Tostmann
(86) Internationale Anmeldenummer: PCT/EP2011/069236
(87) Internationale Veröffentlichungsnummer: WO 2012/059512

(56) Entgegenhaltungen:
- EP-A1- 1 179 535
- EP-A2- 1 179 536

## Beschreibung

Die Erfindung betrifft ein Verfahren zur Gewinnung und Reinigung von Phytosterolen und/oder von Tocopherolen aus Rückständen einer Destillation von Estern pflanzlicher Öle gemäß Patentanspruch 1 oder 4.

Die Hauptquellen von Phytosterolen sind heutzutage Rückstände aus der Tall-ölverarbeitung sowie Dämpferdestillate aus der Pflanzenöl-Raffination, wobei auf Basis dieser Rohstoffe einige Verfahrenspatente existieren. Eine weitere, bislang kaum erschlossene Quelle zur Gewinnung von Phytosterolen und Tocopherolen stellen Destillationsrückstände aus der Pflanzenölmethylesterherstellung für das Einsatzgebiet Biodiesel (FAME) dar. Entsprechend wenige Verfahren sind bekannt.

Grundsätzlich ist bei Destillationsrückständen aus der Pflanzenölmethylesterherstellung zu beachten, dass die Matrix an Begleitkomponenten und Verunreinigungen, die sich im Hinblick auf erzielbare Ausbeuten und Reinheiten störend auf den Sterol- und Tocopherolgewinnungsprozess auswirken können, eine andere als bei Dämpferdestillaten ist. Genannt seien an dieser Stelle exemplarisch Phosphatide, farbgebende Komponenten, angereicherte langkettige Fettsäuremethylester und Polymerisationsprodukte aus der Destillation, die sich im Rückstand wiederfinden. Insofern sind auf die Behandlung von Dämpferdestillaten zugeschnittene Verfahren nicht mit befriedigenden Resultaten auf Destillationsrückstände anwendbar.

In der EP 0 656 894 B2 wird ein Verfahren beschrieben, welches die parallele Gewinnung von sterol- bzw. tocopherolhaltigen Phasen aus Rückständen aus der Rapsölmethylester (RME) -Herstellung ermöglicht. Das Verfahren ist gekennzeichnet durch eine einstufige basisch katalysierte Umesterung mit 50 Gew.-% - 60 Gew.-% eines niederen Alkohols, vorzugsweise Methanol, bei Temperaturen von 60°C - 90°C mit 0,8 Gew.-% - 1,5 Gew.-% Katalysator, vorzugsweise Natriummethylat, gefolgt von einer destillativen Abtrennung des überschüssigen Alkohols und einem Abtrennen der katalysatorhaltigen Glycerinphase. Durch Ansäuern bis zum Neutralpunkt und eine anschließende Wasserwäsche werden in der Esterphase verbliebene Katalysator- und Glycerinreste sowie die gebildeten Alkaliseifen entfernt. Anschließend wird aus der sterol-und tocopherolhaltigen Esterphase der Alkylester destillativ abgetrennt. Aus dem Destillationsrückstand können die Sterole mittels Kristallisation von den Tocopherolen getrennt werden, das Sterolkristallisat wird mit Methanol gewaschen.

Die Kristallisation der Sterole aus einer weitgehend alkylester- und alkoholfreien Matrix verbunden mit den nicht optimalen Umsätzen der Sterolester in der einstufigen Umesterung bewirkt jedoch unzureichende Ausbeuten und Reinheiten der mittels dieses Verfahrens gewonnenen Sterole.

Eine Weiterentwicklung stellt ein in der EP 1 179 535 (2001) sowie in der EP 1 179 536 (2001) beschriebenes Verfahren dar. Sterolreiche Rückstände aus der Destillation umgeesterter Öle pflanzlicher Herkunft (FAME) werden einer zweistufigen basisch katalysierten Umesterung mit kurzkettigen Alkoholen, vorzugsweise Methanol, bei Temperaturen im Bereich von 115°C- 145°C unterzogen. In der ersten Stufe mit 0,5 Gew.-% - 1,8 Gew.-% Katalysator und 5 Gew.-% - 40 Gew.-% Methanol erfolgt eine weitgehende Umsetzung der Partialglyceride zu Fettsäurealkylestern, während in der zweiten Stufe unter verschärften Bedingungen mit 1,8 Gew.-% - 6 Gew.-% Katalysator und 40 Gew.-% - 80 Gew.-% Methanol die Umwandlung von Sterolestern in freie Sterole und Fettsäurealkylester erfolgt. Kennzeichnend für das Verfahren gemäß den vorgenannten Druckschriften ist ferner, dass dort nach jeder Stufe der basische Katalysator mittels Säurezugabe neutralisiert, der überschüssige Alkohol abgeflasht und dann Katalysator und gebildetes Reaktionsglycerin durch Wasserwäsche abgetrennt werden müssen. Zudem muss der Fettsäurealkylester zur Aufkonzentrierung der Sterole im Gemisch nach der ersten Stufe abdestilliert werden. Die freien Sterole werden im Anschluss an die Umesterung durch Abkühlen des Ansatzes auf ca. 20°C auskristallisiert und das so gewonnene Kristallisat mittels einer nicht näher beschriebenen Wäsche mit Lösemittel gereinigt. Die Reinheit der so gewonnenen Sterole wird mit > 90% angegeben, die Ausbeute ist trotz Mutterlaugenrückführung bei der Kristallisation - mit etwas über 50% jedoch nicht befriedigend.

Nachteilig an den Verfahren, wie in der EP 1 179 535 sowie in der EP 1 179 536 beschrieben, ist zudem, dass das Verfahren hohe Umesterungstemperaturen Druckreaktor, lange Reaktionszeiten von über 4 - 8 Stunden, hohe Alkohol-und Katalysatordosierungen, ein Abflashen und Neudosieren des Alkohols, Zugabe von Säure zur Neutralisation des Katalysators und ein Abdestillieren des Fettsäurealkylesters - um dann später solchen wieder als Lösemittel zur Phasentrennung / Unterstützung der Kristallisation zuzugeben - erfordert. Dies alles bedingt hohe Betriebskosten sowie eine aufwendige und komplizierte Verfahrensführung. Zudem ist das Verfahren nicht auf eine parallele Gewinnung einer tocopherolreichen Phase ausgelegt.

Ein weiteres, anderes Verfahrenskonzept wird in EP 1 226 157 (2000) verfolgt. Nach einer einstufigen basisch katalysierten Umesterung eines Rückstandes der Methylesterdestillation erfolgt ohne weiteren Flash- oder Destillationsschritt eine Wasserzugabe in den Rohester, der entsprechend noch Katalysator und eine für das Verfahren erforderliche Menge Methanol enthält. Es bilden sich zwei Phasen aus, wobei die untere wässrige Phase, die auch Methanol und Katalysator enthält, abgetrennt und dann die obere ölige Phase, die Methylester sowie freie und veresterte Sterole enthält, auf Temperaturen vorzugsweise zwischen 1°C - 20°C abgekühlt wird. Die dabei in der Ölphase gebildeten Sterolkristalle werden abgetrennt und zur Reinigung einer Umkristallisation in Methanol sowie einer anschließenden Trocknung unterzogen. Die Zugabe von Wasser in Anwesenheit des Methanols soll eine höhere Reinheit der gewonnenen Sterole bewirken, die trotz Umkristallisation aber nicht über 70% hinausgeht. Selbst wenn eine verfahrensgemäß notwendige Abtrennung hochschmelzender Fettsäuremethylester aus dem umgeesterten Ansatz mit einem Methylestergehalt >20% vorgeschaltet wird, übersteigt die Sterol-Reinheit dort nicht 90% und die Ausbeute nicht 70%.

Die dort vergleichsweise hohe Methanoldosierung von über 100 Gew.-%, bezogen auf den Destillationsrückstand und die verfahrensgemäß hohe Wasserdosierung von 55% und mehr, bezogen auf die im Ansatz vorhandene Methanolmenge, die dort notwendig sind, da sich sonst keine schwere Phase ausbildet, führen zu hohen Betriebskosten. Die Abtrennung der schwereren Wasserphase geht verfahrensgemäß der Abtrennung der Sterolkristalle aus der öligen Phase voraus, was einen zusätzlichen Verfahrensschritt erfordert. Die beiden zusätzlich notwendigen Kristallisationsschritte, nämlich eine Vorabtrennung der hochschmelzenden Methylester und eine Umkristallisation des Sterolkristallisats, verschlechtern ebenfalls die Ökonomie des Verfahrens. Zudem ist auch dieses Verfahren nicht auf die parallele Gewinnung von Tocopherolen ausgerichtet.

Von dem Effekt des Auskristallisierens von Sterolen aus einer Fettsäurealkylester-Alkylalkohol-Matrix im Anschluss an eine sauer katalysierte Veresterung von Pflanzenöl-stämmigen Dämpferdestillaten durch Zugabe von ausreichend Wasser und Kühlung des Ansatzes auf unter 40°C wird bereits in der US 3,335,154 berichtet. In einem ersten Verfahrensschritt werden dort die im Ausgangsmaterial vorhandenen Fettsäuren zusammen mit den Partialglyceriden und Sterolester komplett verseift, die Fettsäurealkaliseifen anschließend wieder durch Säurezugabe gespalten, um dann sauer katalysiert die Veresterung der freigesetzten Fettsäuren zu Methylestern durchzuführen. Durch Zugabe von 5 Gew.-% - 60 Gew.-% Wasser in den Reaktionsansatz und Kühlung auf eine Temperatur zwischen 0°C - 40°C kristallisieren die Sterole aus. Das Kristallisat wird erfindungsgemäß aus der Suspension abgetrennt und durch eine Wäsche mit polaren Lösemitteln gereinigt.

Durch die Schritte Verseifung/Seifenspaltung wird der Nachteil der wesentlich schlechteren Reaktionskinetik einer sauer katalysierten Umesterung im Vergleich zu einer basisch katalysierten umgangen, erkauft durch einen verfahrensgemäß sehr hohen Säure-/Base-Bedarf (20 Gew.-% einer 50%-igen Natronlauge und eine entsprechend überstöchiometrische Menge an HCl). Ferner werden für Verseifung und Veresterung insgesamt 120 Gew.-% Methanol bezogen auf das Ausgangsmaterial eingesetzt. Für Rückstände der Pflanzenölmethylesterherstellung, die in der Regel nur geringste Mengen an Fettsäuren jedoch hohe Gehalte an Methylestern aufweisen, ist dieses Verfahren im Vergleich zur basisch katalysierten Umesterung aufwendig und unökonomisch.

Das dortige Verfahren nutzt den Effekt der erleichterten Phasentrennung in einer Suspension/Emulsion aus Wasserphase, Methylesterphase und Sterolkristallen im sauren Milieu, dennoch ist die Reinheit der Sterolkristalle auch nach intensiver Wäsche mit polaren Lösemitteln nicht ausreichend, weswegen erfindungsgemäß eine zusätzliche Umkristallisation bzw. Lösemittelextraktion mit Hexan nachgeschaltet werden muss.

Ferner wird in der US 5,424,457 ein neueres Verfahren zur Gewinnung von Sterolen ausgehend von Dämpferdestillaten beschrieben. Es ist gekennzeichnet durch eine Alkylzinn-, insbesondere Dibutylzinnoxid, -katalysierte Um-/Veresterung der Sterolester, Partialglyceride und Fettsäuren mit Methanol bei Temperaturen von 150°C bis 240°C, beispielsweise bei 200°C, unter Zugabe von Glycerin, gefolgt von einer destillativen Abtrennung des Überschuss-Methanols und des Reaktionswassers und einer Filtration des Ansatzes bei 100°C zur Abtrennung von Nebenreaktionsprodukten bzw. ausgefallenen Katalysatoranteilen. Nach Abtrennung der katalysatorhaltigen Glycerinphase wird die verbliebene sterolhaltige Filtratphase bei 70°C wiederum mit ca. 16% einer Methanol/Wassermischung (3:1) versetzt. Unter Kühlung auf 25°C kristallisieren dann die Sterole in der Methylester/Methanol/Wasser-Matrix aus. Das Sterolkristallisat wird abfiltriert und unter Redispergierung intensiv mit Lösemittel, nämlich mit auf 5°C gekühlten Heptan gewaschen.

Da das Verfahren gemäß der US 5,424,457 ausgehend von Dämpferdestillat als sterolhaltigem Rohstoff nicht auf die Integration in eine Biodiesel-Anlage ausgerichtet ist, findet eine Katalysatorgattung Verwendung, die in der FAME-Herstellung aufgrund der Kosten und Reaktionsbedingungen nachteilig ist. Insbesondere das im Patent dargestellte Problem einer potentiellen Zinn-Belastung des Endproduktes spricht gegen eine Verwendung der mittels dieses Verfahrens gewonnenen Sterole im Food-Sektor. Unvorteilhaft sind die gemäß Ausführungsbeispiel zur Wäsche des Kristallisats erforderlichen großen Mengen gekühlten Lösemittels von über 1000% bezogen auf die gewonnenen Sterole. Bemerkenswert ist die mit 98% angegebene Reinheit der Sterole, jedoch zeigen die weiteren Ausführungen, dass die erzielbaren Ausbeuten zugunsten niedriger Zinn-Gehalte im Endprodukt auf deutlich unter 70% sinken. Nachteilig ist weiterhin das Handling bzw. die Entsorgung der, nach dem Verfahrensschritt einer Entfernung des Überschuss-Methanols, abfiltrierten Nebenreaktionsprodukte sowie der stark mit Zinn belasteten Glycerinphase anzusehen.

Der Erfindung liegt die Aufgabe zugrunde unter Vermeidung der vorgenannten Nachteile ein einfaches und kostengünstiges Verfahren zur Gewinnung von freien Sterolen und/oder Tocopherolen, jeweils in hoher Reinheit und in jeweils hoher Ausbeute aus Destillationsrückständen einer Biodiesel-Produktion (= FAME-Produktion) anzugeben, das sich durch wenige Verfahrensschritte und die Verwendung von in FAME-Anlagen üblichen Stoffen als Reaktanten und über eine somit gegebene volle Implementierung in eine FAME-Anlage als besonders ökonomisch darstellt.

Diese Aufgabe wird durch ein Verfahren gemäß Patentanspruch 1 oder Patentanspruch 4 gelöst.

Gemäß einer bevorzugten und besonders vorteilhaften Ausführungsform der Erfindung erfolgt ferner nach der zweiten Umesterungsstufe ein Zugeben von Wasser zu dem Reaktionsgemisch, um ein Mehrphasensystem zu erzeugen. Im Anschluss daran wird erfindungsgemäß ein gleichzeitiges oder sequentielles Trennen der Phasen des Mehrphasensystems in
- eine im Wesentlichen sterolhaltige Phase;
- eine im Wesentlichen glycerin- und methanolhaltige wässrige Phase; und
- eine tocopherolhaltige Methylesterphase; sowie
- ein Gewinnen von Phytosterolen aus der sterolhaltigen Phase; und/oder
- gegebenenfalls ein Gewinnen von Tocopherolen aus der tocopherolhaltigen Methylesterphase
durchgeführt.

Ein wesentlicher Punkt der Erfindung besteht darin, dass das erfindungsgemäße Verfahren aus einer zweistufigen basisch katalysierten Umesterung eines Fettsäuremethylester-Destillationsrückstandes (= FAME-Destillationsrückstand) aus der Biodieselherstellung mit einer Zwischenabtrennung einer bei der Umesterung anfallenden Glycerinphase zur Vervollständigung des Glyceridumsatzes in der zweiten Reaktionsstufe ausgeführt wird, ohne dass Methanol oder Katalysator durch Flashen, Destillieren oder eine Wäsche entfernt werden müssen.

Erfindungsgemäß und in vorteilhafter Weise kann somit ein Reaktionsgemisch der ersten Umesterungsstufe unmittelbar in einer zweiten Umesterungsstufe weiterverarbeitet werden, wobei es vor einem Schritt, bei welchem die Phasen des erfindungsgemäß erzeugten Mehrphasensystems ohnehin getrennt werden, nicht notwendig ist, Methanol oder Katalysator aus dem Reaktionsgemisch zu entfernen. Mit dieser Verfahrensweise ist es somit erfindungsgemäß nicht nur möglich sehr wirtschaftlich und einfach zu arbeiten, sondern auch die Umsetzungsgrade sind ferner so gut, dass bei einer Weiterbehandlung des Reaktionsgemisches bisher unerreichte Ergebnisse in Ausbeute und Reinheit erreicht werden können.

Die nach der ersten Umesterungsstufe anfallende Glycerinphase kann in vorteilhafter Weise unmittelbar einer einem Biodieselherstellungsprozess angegliederten Glyceringewinnung zugeführt werden.

Das erfindungsgemäße Verfahren wird ferner so durchgeführt, dass die erste und/oder die zweite Umesterungsstufe bei einer Temperatur im Bereich von Raumtemperatur (= 25°C) bis 88°C, vorzugsweise im Bereich von 40°C bis 75°C und besonders bevorzugt im Bereich von 55°C bis 70°C, sowie ferner insbesondere bei Normaldruck, durchgeführt wird. Diese Ausführungsform der Erfindung ermöglicht eine energiesparende und kosteneffiziente Durchführung des Verfahrens, da hohe Heizkosten vermieden werden und die jeweiligen Umesterungsreaktionen unter Anderem bei Normaldruck durchgeführt werden können, so dass erfindungsgemäß auf teure Druckreaktoren sowie auf eine aufwendige und teure Erzeugung und Aufrechterhaltung der Temperaturen und Drücke, wie sie beim Stand der Technik notwendig sind, verzichtet werden kann.

Ferner trägt die niedrige Umsetzungstemperatur während der ersten und/oder der zweiten Umesterungsstufe zu einer deutlichen Reduzierung der Betriebskosten gegenüber bekannten Verfahren bei und verbessert somit maßgeblich auch die Ökonomie des Verfahrens gegenüber bisher üblichen Verfahren.

Ein weiterer Vorteil der erfindungsgemäß drucklos durchführbaren Umesterungen liegt ferner darin, dass auch aufwendige Sicherheitsmaßnahmen, die im Falle der Verwendung von Druckbehältern notwendig sind, unter Anwendung des erfindungsgemäßen Verfahrens entfallen können, da alle Arbeiten bei normal- bzw. Atmosphärendruck, sowie, bedingt durch die niedrigen Reaktionstemperaturen, energieeffizient und zeitsparend, durchgeführt werden.

Erfindungsgemäß wird die erste Umesterungsstufe mit einem Gehalt an basischem Katalysator, bevorzugt Natriummethylat, aber beispielsweise auch Natriumhydroxid (NaOH) oder Kaliumhydroxid (KOH), im Bereich von 0,1% bis 0,3% bevorzugt im Bereich von 0,18% bis 0,22% sowie mit einem Gehalt an Methanol im Bereich von 12% bis 18%, bevorzugt im Bereich von 14% bis 16% und die zweite Umesterungsstufe mit einem Gehalt an Katalysator im Bereich von 0,5% bis 1% , bevorzugt im Bereich von 0,6% bis 0,8% sowie mit einem Gehalt an Methanol im Bereich von 20% bis 38%, bevorzugt im Bereich von 34% bis 36%, durchgeführt, wobei die Zugabemenge an basischem Katalysator auf eine Zugabe an Natriummethylat normiert und im Hinblick auf eine Verwendung anderer basischer Katalysatoren gegebenenfalls anzupassen ist. Aufgrund dieser im Bezug auf bekannte Verfahren sehr geringen notwendigen Katalysator- und Methanolzugaben zu den einzelnen Umesterungsstufen ist das erfindungsgemäße Verfahren besonders kostengünstig und recyclingfreundlich zu betreiben, da beispielsweise nur geringe Methanolmengen einer Methanolrückgewinnung zugeführt werden müssen. Im Übrigen ist der erfindungsgemäß verwendete basische Katalysator unter Umwelt- sowie auch lebensmittelrelevanten Aspekten völlig unproblematisch einsetz- und recyclebar, wobei in vorteilhafter Weise, anders, als beispielsweise in vorgenannter US 5,424,457 keine Schwermetallbelastung in den erzeugten Produkten, hier Phytosterolen und/oder Tocopherolen, zu befürchten sind.

Gemäß einer bevorzugten Ausführung wird beim Zugeben von Wasser dieses mit einer Menge im Bereich von 15% bis 25%, vorzugsweise von 18% bis 22% und besonders bevorzugt im Bereich von 19,5% bis 20,5%, jeweils bezogen auf die Masse eines Gesamtansatzes, zugegeben, um insbesondere ein Massenverhältnis von Sterol : Fettsäuremethylestern : Methanol : Wasser von im Wesentlichen 1 : 2,5- 3 : 2,2 - 2,5 : 0,8 - 1,2 einzustellen.

Die Zugabe von Wasser zu dem Reaktionsgemisch, die erfindungsgemäß nach der zweiten Umesterungsstufe erfolgt, ermöglicht es auf besonders einfache Weise Stoffe, insbesondere aus einer sterolhaltigen Phase des umgeesterten Ansatzes, zu entfernen, welche eine Kristallisation der Sterole behindern würden. So wird durch die Zugabe von Wasser in dem Reaktionsgemisch vorhandenes Glycerin, Katalysator sowie Verunreinigungen aus dem Destillationsrückstand abgetrennt, wobei die genannten Stoffe in die Wasserphase übergehen. Das zugegebene Wasser entzieht darüber hinaus dem Reaktionsansatz weitgehend das noch vorhandene Methanol, wodurch die Löslichkeit der Sterole in der Methylesterphase stark abnimmt und diese auskristallisieren.

Darüber hinaus wurde im Zuge der Zugabe von Wasser zu dem Reaktionsgemisch überraschend festgestellt, dass bei Erreichen einer bestimmten Wasserkonzentration ein spontanes, sehr vollständiges Auskristallisieren der Sterole bereits bei der Reaktionstemperatur zu beobachten ist, wobei sich gleichzeitig ein 3-Phasensystem, bestehend aus einer Fettsäuremethylesterphase, einer Wasserphase sowie aus Sterolkristallen ausbildet, wobei die jeweilige Dichte der drei Phasen in der vorgenannten Reihenfolge ansteigt. So hat sich herausgestellt, dass insbesondere das Zugeben im vorgenanntem Mengenverhältnis von Sterol : Fettsäuremethylestern : Methanol : Wasser von im Wesentlichen 1 : 2,5 - 3 : 2,2 - 2,5 : 0,9 - 1,1 besonders wirkungsvoll ist, um eine klare Trennung der drei Phasen zu erreichen, wodurch eine Weiterverarbeitung des Reaktionsgemisches stark vereinfacht wird, was sich wiederum äußerst positiv auf die Verfahrensökonomie, insbesondere in Bezug auf eine energie- und zeitsparende Umsetzung der Ausgangsprodukte sowie eine Gewinnung der gewünschten Phytosterole und Tocopherole auswirkt.

Gemäß einer weiteren Ausführungsform der Erfindung wird im Zuge der ersten Umesterungsstufe nach einem Einmischen von Methanol und Katalysator Glycerin in einer Menge im Bereich von 0,2% bis 7,2%, bevorzugt im Bereich von 0,5% bis 6,0% und besonders bevorzugt im Bereich von 1,0% bis 5,5%, jeweils bezogen auf die Masse des Gesamtansatzes, zugegeben. Durch dieses erfindungsgemäße Beimischen von Glycerin zu dem Gesamtansatz verbessert sich die spätere Phasentrennung und es werden Verunreinigungen in vorteilhafter Weise besser in die schwere Glycerinphase ausgetragen.

Des Weiteren wird im Zuge einer bevorzugten Ausführungsform der Erfindung der Destillationsrückstand aus einer Umesterung von pflanzlichen Ölen durch eine Zugabe von Fettsäuremethylester vor der ersten und/oder zweiten Umesterungsstufe so eingestellt, dass eine Löslichkeit der Sterole während der Umesterung gewährleistet ist und erhalten bleibt, so dass diese erfindungsgemäß nicht schon bei der ersten und/oder zweiten Umesterungsstufe unkontrolliert ausfallen, sondern kontrolliert in Lösung bleiben.

Im Übrigen werden die Parameter der Umesterung, insbesondere die Dosierung des basischen Katalysators sowie die Reaktionstemperaturen, erfindungsgemäß so gewählt, dass ein maximaler Umsatz der Partialglyceride bzw. Sterolester bei weitgehender Schonung der im Destillationsrückstand vorhandenen Tocopherole erreicht wird.

Gemäß einer weiten vorteilhaften Ausführungsform wird das Reaktionsgemisch, insbesondere nach dem Zugeben von Wasser in oben definierten Massenverhältnissen, durch Mischen zu einer Emulsion bzw. einer Suspension homogenisiert. Durch dieses konstante Durchmischen des Reaktionsgemisches wird ein Absedimentieren von nach der Wasserzugabe bereits gebildeten Sterolkristallen vermieden, wobei durch die Homogenisierung der Kristallisationsprozess der Phytosterolkristalle und eine für die Weiterverarbeitung optimierte Kristallbildung unterstützt werden.

Des Weiteren hat es sich als vorteilhaft erwiesen, die homogenisierte Emulsion bzw. Suspension auf eine Temperatur im Bereich von 5°C bis 35°C, vorzugsweise im Bereich von 10°C bis 30°C und besonders bevorzugt im Bereich von 15°C bis 25°C abzukühlen, wodurch eine anschließende Phasenseparation deutlich erleichtert wird. Darüber hinaus kann die Kristallstruktur der gewünschten Phytosterolkristalle durch das Einhalten einer Reifezeit deutlich verbessert werden, was sich wiederum auf verbesserte Filtrationseigenschaften der Kristalle und auch Ausbeuten an Kristallen positiv bemerkbar macht. Die Reifezeit liegt erfindungsgemäß, insbesondere im Bereich von 1 Stunde bis 48 Stunden, bevorzugt im Bereich von 2 Stunden bis 36 Stunden und besonders bevorzugt im Bereich von 4 Stunden bis 12 Stunden.

Das Trennen der Phasen wird erfindungsgemäß mittels einer Filter-, Sieb- und/oder Dekantierzentrifuge durchgeführt, wobei bevorzugt eine Filterzentrifuge zum Einsatz kommt. Durch den Einsatz einer Filter- oder Dekantierzentrifuge kann praktischerweise ein Filterkuchen mit einer deutlich niedrigeren Restfeuchte erhalten werden, als dies beispielsweise bei einer Differenzdruckfiltration möglich wäre.

Des Weiteren ist auch ein 3-Phasendekanter gut geeignet, um das erfindungsgemäße Mehrphasensystem aus sterolhaltiger Phase, glycerin- und methanolhaltiger Phase sowie tocopherolhaltiger Phase zu trennen, wobei sich die Sterolkristalle enthaltende Phase, respektive die Sterolkristalle, als schwerste Phase ausbilden und über den 3-Phasendekanter gut abtrennen bzw. voreindicken lässt, während gleichzeitig die Fettsäuremethylesterphase und die glycerin- und methanolhaltige Wasserphase getrennt gewonnen werden können.

Die Abtrennung der Sterolkristalle mittels einer diskontinuierlich arbeitenden Filterzentrifuge bietet hierbei zudem die Möglichkeit, gleich im Anschluss an die Filtration eine Kuchenwaschung durchzuführen.

Die sterolhaltige Phase, die im Wesentlichen Sterolkristalle aufweist, wird im weiteren Verlauf mit Methanol gewaschen, wobei die Menge an Methanol im Bereich von 50% bis 800%, vorzugsweise im Bereich von 125% bis 700% und besonders bevorzugt im Bereich von 200% bis 550%, jeweils bezogen auf die Masse der Sterolkristallphase, liegt. Durch die Anwendung dieser einfach durchzuführenden Methanolwäsche ist es möglich, eventuell verbliebene Reste an Fettsäuremethylester- und Wasserphase, die an den Sterolkristallen möglicherweise verblieben sind, zu beseitigen und die Sterolkristalle auf diese Weise effizient von der sich aus Fettsäuremethylester- und Wasserphase bestehenden Zwickelflüssigkeit zu trennen und auf diese Weise zu reinigen. Das aus dieser Methanolwäsche resultierende Waschmethanol kann im Anschluss ohne eine weitere Aufreinigung, insbesondere ohne Rektifikation, dem Prozess der Biodieselherstellung zugeführt werden.

Des Weiteren wird die Anwendung eines Verfahrens offenbart, das eine Herstellung hochreiner Sterolkristalle ermöglicht, wobei betont sei, dass dieses offenbarte Reinigungsverfahren explizit auch generell zur Reinigung von Sterolkristallphasen und/oder Sterolkristallen hervorragend geeignet ist.

Demgemäß kann der Methanolwäsche optional auch eine Verdrängungswäsche am Sterol-Filterkuchen mit Fettsäuremethylester, vorzugsweise, aber nicht ausschließlich der gleichen Art, aus dem der Destillationsrückstand stammt, also beispielsweise Rapsmethylester sofern der Destillationsrückstand aus der Rapsmethylester-Produktion verarbeitet wird, vorangestellt werden. Zusätzlich oder alternativ können für eine solche Verdrängungswäsche auch andere Fettsäuremethylester, wie beispielsweise Soja- und/oder Sonnenblumen- und/oder Kokos- und/oder Palm- und/oder Baumwollsaatöl- und/oder Maiskeimölmethylester verwendet werden, sofern dies gewünscht ist. Eine Verwendung dieser Ester oder Mischungen dieser Ester kann beispielsweise im Hinblick auf Kostenaspekte, jedoch auch im Hinblick auf eine Einstellbarkeit von Lösemitteleigenschaften der für die Verdrängungswäsche eingesetzten Fettsäuremethylester, beispielsweise hinsichtlich eventueller, möglicherweise herkunftsbedingter Verunreinigungen der eingesetzten Rohstoffe, vorteilhaft sein. Durch diese vorhergehende Verdrängungswäsche mit Methylester kann die Qualität des Kristallisats, insbesondere dessen Reinheit und Farbe, weiter deutlich verbessert werden. Der im Vergleich zum Methanol viskosere Methylester ist dabei in der Lage, die aus der Filtration des Reaktionsansatzes im Sterolkristallisat verbliebene Zwickelflüssigkeit und darin enthaltene Verunreinigungen zu verdrängen. Aufgrund der geringeren Polarität des Methylesters ist dieser zudem in der Lage, bestimmte an den Sterolkristallen anhaftende Verunreinigungen zu lösen, die mit der reinen Methanolwäsche nur bedingt zu entfernen sind. Durch die kurze Einwirkzeit der Verdrängungswäsche können die Sterolverluste durch Rücklösen im Methylester auf ein Minimum reduziert werden.

Die vorgenannte Verdrängungswäsche mit Methylester wird bevorzugt mit einem Mengenverhältnis im Bereich von 15% bis 500%, vorzugsweise im Bereich von 75% bis 400% und besonders bevorzugt im Bereich von 100% bis 350%, jeweils bezogen auf die Masse der Sterolkristallphase durchgeführt, um die Reinheit und Farbe der Sterolkristalle auf ein gewünschtes Maß anzupassen.

Das auf diese Weise gewonnene Kristallisat von Phytosterolen kann im Anschluss an die Methanolwäsche unmittelbar getrocknet werden, um auf diese Weise ein rieselfähiges Pulver zu erhalten, das ohne weitere Behandlung, insbesondere ohne die Notwendigkeit einer weiteren Reinigung oder Umkristallisation, verpackt werden kann.

Somit kommt das erfindungsgemäße Verfahren, anders als aus dem Stand der Technik bekannte Verfahren, bei der Gewinnung von Sterolkristallen ohne eine weitere Reinigung, insbesondere ohne eine Umkristallisation oder eine Rekristallisation aus, was wiederum zur besonderen Ökonomie und Effizienz des erfindungsgemäßen Verfahrens gegenüber bereits bekannten gattungsgemäßen Verfahren gemäß dem Stand der Technik beiträgt.

Darüber hinaus können durch die Anwendung des erfindungsgemäßen Verfahrens Phytosterole aus den Destillationsrückständen einer Umesterung pflanzlicher Öle mit einer Reinheit von über 95% mit Ausbeuten von über 80% gewonnen werden, was sowohl hinsichtlich Reinheit als auch hinsichtlich Ausbeute deutlich über aus dem Stand der Technik bekannte Verfahren hinausgeht.

Im Zuge einer weiteren Gewinnung von Tocopherolen aus der tocopherolhaltigen Phase wird die Fettsäuremethylesterphase des Mehrphasensystems, welche die Tocopherole in gelöster Form enthält, vorzugsweise einer Destillation zur Abtrennung der Methylester unterzogen, wodurch es möglich ist den Tocopherolgehalt in der Fettsäuremethylesterphase auf über 10% aufzukonzentrieren, um so eine einfache weitere Aufbereitung der Tocopherole in bekannter Weise zu ermöglichen.

An dieser Stelle sei ferner erwähnt, dass die bei vorgenannter Destillation abgetrennten Fettsäuremethylester wiederum unmittelbar zur Einstellung der Konsistenz des Rückstandes aus der Biodieseldestillation gemäß einem ersten optionalen Verfahrensschritt verwendet werden können. Darüber hinaus ist es möglich, diese Fettsäuremethylester dem bei der Biodieseldestillation gewonnenen Destillat direkt zuzuschlagen, was wiederum die Wirtschaftlichkeit des erfindungsgemäßen Verfahrens weiter verbessert. Diesbezüglich sei auch nochmals darauf hingewiesen, dass die glycerin- und methanolhaltige Wasserphase einer Methanolrückgewinnung in einer Biodieselanlage zugeführt werden kann, wobei das Verfahren aufgrund der erfindungsgemäßen spezifisch geringen Menge an anfallender Wasserphase sehr einfach und kostengünstig durchführbar ist. Erwähnenswert ist an dieser Stelle noch, dass die erfindungsgemäße Wasserzudosierung so gewählt ist, dass sich Kristalle mit einer Größe bilden, die einfach abscheidbar und/oder filtrierbar sind, wobei eine höhere Wasserzudosierung zu kleineren und somit schwerer abscheidbaren bzw. filtrierbaren Kristallen führen würde. Eine Zudosierung von weniger Wasser zu dem Reaktionsgemisch würde im Gegenzug zu einer Abnahme der Dichte der Wasserphase führen, was wiederum schlechtere Ergebnisse bei der Phasentrennung und damit auch eine schlechtere Ausbeute bedeuten würde.

Somit ist das erfindungsgemäße Verfahren in vorteilhafter Weise vollständig in einen Prozess zur Herstellung von Biodiesel implementierbar, wobei lediglich Katalysator, Methanol und Wasser in jeweils gegenüber dem bisherigen Stand der Technik deutlich geringeren Mengen notwendig sind, was zum einen eine kostengünstige Verfahrensführung ermöglicht, und zum anderen eine Methanolrückgewinnung verbilligt. Darüber hinaus ist bei dem erfindungsgemäßen Verfahren weder eine Umkristallisation noch eine Rekristallisation der gewonnen Phytosterolkristalle notwendig, auch kann auf den Einsatz von separat zu regenerierenden Lösemitteln, wie z.B. Aceton, Kohlenwasserstoffen usw., als Waschmedium verzichtet werden, wobei auch die Menge an Waschmedium erfindungsgemäß deutlich niedriger ausfällt als bei anderen Verfahren und das verwendete Waschmethanol in vorteilhafter Weise unmittelbar in einem Prozess zur Biodieselherstellung weiter genutzt werden kann. Des Weiteren kann der Kristallisationsansatz zwar auf Temperaturen bis zu 5°C abgekühlt werden; es ist jedoch erfindungsgemäß nicht zwingend notwendig, den Kristallisationsansatz auf eine Temperatur von unterhalb 20°C abzukühlen. Darüber hinaus ist trotz eines bis zu 20%-igen Fettsäuremethylesteranteils im umgeesterten Ansatz keine Vorabtrennung hochschmelzender Methylester notwendig. Ein weiterer wichtiger Vorteil des erfindungsgemäßen Verfahrens besteht darüber hinaus in dem möglichen einfachen Einsatz eines 3-Phasendekanters, um die Phytosterolkristalle als schwerste Phase aus dem erfindungsgemäßen Mehrphasengemisch abzutrennen. Darüber hinaus ist eine nahezu vollständige Gewinnung der in dem Destillationsrückstand enthaltenen Tocopherole möglich.

Zusammenfassend kann somit festgehalten werden, dass anhand des erfindungsgemäßen Verfahrens, welches insbesondere gekennzeichnet ist durch eine zweistufige basisch katalysierte Umesterung mit einer Glycerinphasenabscheidung nach der ersten Umesterungsstufe und anschließender Sterolkristallisation aus dem Reaktionsgemisch unter Zugabe von Wasser, wobei auf zwischengeschaltete Verfahrensschritte wie Neutralisation, Abdestillieren von Reaktanten bzw. Lösemitteln, Auswaschen von Katalysator verzichtet wird, und welches ferner anhand einer Kombination aus Methylester-Verdrängungswäsche gefolgt von einer Methanolwäsche des Sterolkristallisat-Filterkuchens, unter Einhaltung bestimmter vorgenannter Verfahrensparameter eine Gewinnung von Phytosterolen und Tocopherolen aus Destillationsrückständen aus einer Umesterung von pflanzlichen Ölen, insbesondere aus der pflanzenölbasierten Fettsäuremethylesterherstellung für das Einsatzgebiet Biodiesel mit bislang nicht erreichten Reinheiten und Ausbeuten möglich ist. Ferner lässt das zuvor beschriebene erfindungsgemäße Verfahren die volle Implementierung in eine Anlage zur FAME-Erzeugung zu, wobei in vorteilhafter erfindungsgemäßer Weise die in FAME-Anlagen üblichen Substanzen in optimaler Weise als Reaktanten eingesetzt werden können, weswegen das Verfahren sowohl unter wirtschaftlichen Gesichtspunkten als auch unter Logistikaspekten besonders effektiv und ökonomisch ist.

Weitere Ausführungsformen der Erfindung ergeben sich aus den Unteransprüchen.

Nachfolgend wird die Erfindung anhand von Ausführungsbeispielen näher erläutert.

### 1. Ausführungsbeispiel:

3850 g eines Rückstandes aus der Destillation von Rapsmethylester wurden erfindungsgemäß 1782 g RME beigemischt. Die Analyse des Ansatzes ergab Gehalte von 21,73% Sterolester, 6,21% freie Sterole, 1,68% Tocopherole, 9,8% Glyceride und 44,17 % Methylester.

Der Ansatz wurde auf 65°C temperiert und in einer ersten Umesterungsstufe 37,5 g Na-Methylat (30%ige Lösung in Methanol) sowie 818 g Methanol zugegeben und eingemischt. Nach 50 Minuten Absetzzeit wurden 301,2 g glycerinhaltige Bodenphase abgezogen. Der Umsatz bei den Partialglyceriden lag über 95%.

Für die zweite Umesterungsstufe zur Umwandlung der Sterolester in freie Sterole wurden 150,2 g Na-Methylat (30%ige Lösung in Methanol) sowie 1865,6 g Methanol zugegeben. Die Reaktion erfolgte bei 65°C über 90 Minuten.

Dem Ansatz wurden unter Rühren 1126 g Wasser zugegeben, wobei sich Sterolkristalle bildeten. Die Suspension wurde unter Rühren auf 20°C abgekühlt und dann bei dieser Temperatur einer Reifung unterzogen.

Anschließend wurde die Suspension mittels einer Filterzentrifuge filtriert, der gebildete Kuchen noch in der Zentrifuge einer ersten Wäsche mit 3,5 Liter RME-Destillat und einer zweiten Wäsche mit 10,4 Liter Methanol unterzogen. Nach der Trocknung des methanolfeuchten Filterkuchens resultierten 908 g weißes Sterolpulver mit einem Sterolgehalt von über 98%, was einer Ausbeute (bezogen auf den Gesamtsterolgehalt des Destillationsrückstandes) von über 82% entspricht.

Das Filtrat aus der Filtration der Suspension trennte sich selbsttätig in eine leichte, methylester-, sterol- und tocopherolhaltige und in eine wässrige, methanol- und katalysatorhaltige Phase auf. Auch in der Wasch-RME-Phase waren Sterole und Tocopherole gelöst, während in der Waschmethanolphase keine Tocopherole nachweisbar waren.

In den vereinigten Methylester-Phasen fanden sich 87% der ursprünglich im RME-Destillationsrückstand nachgewiesenen Tocopherole. Nach Destillation der Methylester-Phasen konnte ein Rückstand mit einem Tocopherolgehalt von 11% gewonnen werden, der zur weiteren Aufarbeitung der Tocopherole geeignet ist.

### 2. Ausführungsbeispiel:

3119 g eines Rückstandes aus der Destillation von Rapsmethylester wurden erfindungsgemäß 2324 g RME beigemischt. Die Analyse des Ansatzes ergab Gehalte von 27,2% Sterolester, 5,17% freie Sterole, 1,12% Tocopherole, 8,14% Glyceride und 42,74 % Methylester.

Der Ansatz wurde auf 65°C temperiert und in einer ersten Umesterungsstufe 36,3 g Na-Methylat (30%ige Lösung in Methanol) sowie 873,5 g Methanol zugegeben und eingemischt. Nach 50 Minuten Absetzzeit wurden 319,2 g glycerinhaltige Bodenphase abgezogen. Der Umsatz bei den Partialglyceriden lag über 95%.

Für die zweite Umesterungsstufe zur Umwandlung der Sterolester in freie Sterole wurden145,1 g Na-Methylat (30%ige Lösung in Methanol) sowie 1995,7 g Methanol zugegeben. Die Reaktion erfolgte bei 65°C über 90 Minuten.

Dem Ansatz wurden unter Rühren 1208 g Wasser zugegeben, wobei sich Sterolkristalle bildeten. Die Suspension wurde unter Rühren auf 20°C abgekühlt und dann bei dieser Temperatur einer Reifung unterzogen.

Anschließend wurde die Suspension mittels einer Filterzentrifuge filtriert, der gebildete Kuchen noch in der Zentrifuge einer ersten Wäsche mit 2,4 Liter RME und einer zweiten Wäsche mit 10,4 Liter Methanol unterzogen. Nach der Trocknung des methanolfeuchten Filterkuchens resultierten 956 g weißes Sterolpulver mit einem Sterolgehalt von über 98%, was einer Ausbeute (bezogen auf den Gesamtsterolgehalt des Destillationsrückstandes) von 80% entspricht.

Sofern gewünscht, kann die aufkonzentrierte Methylesterphase im Rahmen einer erneuten Umesterung und Kristallisation weiterverwendet werden.

Gemäß einem weiteren exemplarischen Ausführungsbeispiel des erfindungsgemäßen Verfahrens wird in einem ersten Schritt die Konsistenz eines Destillationsrückstandes aus der Umesterung von pflanzlichen Ölen zur Herstellung von Biodiesel durch die Zugabe von Fettsäuremethylester für eine weitere Verarbeitung in einer ersten Umesterungsstufe eingestellt, wobei so viel Fettsäuremethylester zu dem Destillationsrückstand zugegeben wird, dass die Löslichkeit der in dem Destillationsrückstand enthaltenen Sterole während der folgenden Umesterungen erhalten bleibt. Dann wird in einer ersten Umesterungsstufe mit einem Gehalt von 0,2% Katalysator, nämlich Natriummethylat, und 15% Methanol eine Umsetzung der Partialglyceride aus dem Destillationsrückstand vorgenommen, wobei nach der Zugabe des Katalysators sowie des Methanols ferner 1% bis 5% Glycerin zur Verbesserung einer späteren Phasentrennung hinzudosiert werden. Als nächstes wird die Glycerinphase, die sich in dem Reaktionsgemisch gebildet hat, abgetrennt, wobei Verunreinigungen, insbesondere Phosphatide, in die Glycerinphase ausgetragen werden. Im Anschluss daran wird eine zweite Umesterungsstufe mit dem verbliebenen Reaktionsgemisch durchgeführt, wobei in dem Reaktionsgemisch nunmehr 0,8% Katalysator sowie 35% Methanol enthalten sind oder gegebenenfalls auf diesen Anteil ergänzt werden. Eine vorhergehende Abtrennung von Katalysator und Methanol nach der ersten Umesterungsstufe ist hierbei nicht notwendig. Nach der zweiten Umesterungsstufe, die ebenso, wie die erste Umesterungsstufe bei einer Temperatur von 65°C unter Atmosphärendruck durchgeführt wird, werden zur Bewirkung einer Kristallisation der in dem Reaktionsgemisch enthaltenen Phytosterole ca. 20 Vol.-% Wasser zu dem Reaktionsgemisch zugegeben, wodurch sich ein Mehrphasensystem aus Sterolkristallphase, einer glycerin- und methanolhaltigen wässrigen Phase sowie einer tocopherolhaltigen Fettsäuremethylesterphase ergibt. Aus diesem Mehrphasensystem werden nunmehr durch Zentrifugation sowie Filtration der Suspension die Phytosterolkristalle abgetrennt und mit einer 1- bis 3-fachen Gewichtsmenge an Rapsmethylester im Rahmen einer Verdrängungswäsche gewaschen, der eine weitere Wäsche mit Methanol, und zwar in einer 2- bis 5-fachen Gewichtsmenge des Kristallisats folgt. Nach dieser Methanolwäsche wird das Kristallisat getrocknet und einer Verpackung zugeführt. Eine weitere Aufbereitung der verbliebenen Reaktionsmischung erfolgt durch eine Trennung der wässrigen sowie der Methylesterphase, wobei die Methylesterphase zur Aufkonzentrierung des Tocopherolgehalts destilliert und die Methylester auf diese Weise weitgehend abgetrennt werden. Die verbliebene tocopherolreiche Methylesterphase wird sodann einer Weiterverarbeitung und einer Gewinnung der Tocopherole zugeführt.

An dieser Stelle sei darauf hingewiesen, dass alle oben beschriebenen Teile für sich alleine gesehen und in jeder Kombination als erfindungswesentlich beansprucht werden.

## Patentansprüche

1. Verfahren zur Gewinnung von Phytosterolen und/oder Tocopherolen aus Rückständen einer Destillation von Estern pflanzlicher Öle
**gekennzeichnet durch**
eine zweistufige basische Umesterung mit einer zwischengeschalteten Abtrennung von Glycerinphase,
wobei
- in einer ersten basischen Umesterungsstufe eine Umsetzung von in den Destilliations-Rückständen enthaltenen Partialglyceriden durchgeführt wird;
- aus einem aus der ersten basischen Umesterungsstufe unmittelbar resultierenden Reaktionsgemisch Glycerinphase abgetrennt wird;
und
- in einer zweiten basischen Umesterungsstufe eine Umsetzung von in dem Reaktionsgemisch enthaltenen Sterolestern durchgeführt wird,
wobei das Verfahren aus einer zweistufigen basisch katalysierten Umesterung eines Fettsäuremethylester-Destillationsrückstandes aus der Biodieselhersteflung mit einer Zwischenabtrennung einer bei der Umesterung anfallenden Glycerinphase zur Vervollständigung des Glyceridumsatzes in der zweiten Reaktionsstufe ausgeführt wird, ohne dass Methanol oder Katalysator durch Flashen, Destillieren oder eine Wäsche entfernt werden.

2. Verfahren nach Anspruch 1, ferner **gekennzeichnet durch**
- ein Zugeben von Wasser zu dem Reaktionsgemisch nach der zweiten Umesterungsstufe zur Erzeugung eines Mehrphasensystems;
- ein gleichzeitiges oder sequenzielles Trennen der Phasen des Mehrphasensystems in
- eine im Wesentlichen sterolhaltige Phase;
- eine im Wesentlichen glycerin- und methanolhaltige wässrige Phase; und
- eine tocopherolhaltige Methylesterphase;
- ein Gewinnen von Phytosterolen aus der sterolhaltigen Phase; und/oder
- gegebenenfalls ein Gewinnen von Tocopherolen aus der tocopherolhaltige Methylesterphase.

3. Verfahren nach einem der vorhergehenden Ansprüche, **dadurch gekennzeichnet, dass**
die erste und/oder die zweite Umesterungsstufe bei einer Temperatur im Bereich von Raumtemperatur (25 °C) bis 88 °C, vorzugsweise im Bereich von 40 °C bis 75 °C und besonders bevorzugt im Bereich von 55 °C bis 70 °C, sowie ferner insbesondere bei Normal bzw. Atmosphärendruck, durchgeführt wird.

4. Verfahren zur Gewinnung von Phytosterolen und/oder Tocopherolen aus Rückständen einer Destillation von Estern pflanzlicher Öle, vorzugsweise aus Destillationsrückständen aus einer Umesterung von pflanzlichen Ölen, insbesondere aus der pflanzenölbasierten Fettsäutemethtylesterhtersteltung für das Einsatzgebiet Biodiesel (FAME),
**gekennzeichnet durch**
eine zweistufige basische Umesterung mit einer zwischengeschalteten Abtrennung von Glycerinphase,
wobei
- in einer ersten basischen Umesterungsstufe eine Umsetzung von in den Destillations-Rückständen enthaltenen Partialglyceriden durchgeführt wird;
- aus einem aus der ersten basischen Umesterungsstufe unmittelbar resultierenden Reaktionsgemisch Glycerinphase abgetrennt wird;
und
- in einer zweiten basischen Umesterungsstufe eine Umsetzung von in dem Reaktionsgemisch enthaltenen Sterolestern durchgeführt wird,
und wobei die erste und/oder die zweite Umesterungsstufe bei einer Temperatur im Bereich von Raumtemperatur (25 °C) bis 88 °C, vorzugsweise im Bereich von 40 °C bis 75 °C und besonders bevorzugt im Bereich von 55 °C bis 70 °C, sowie ferner insbesondere bei Normal bzw. Atmosphärendruck, durchgeführt wird.

5. Verfahren nach Anspruch 4, ferner **gekennzeichnet durch**
- ein Zugeben von Wasser zu dem Reaktionsgemisch nach der zweiten Umesterungsstufe zur Erzeugung eines Mehrphasensystems;
- ein gleichzeitiges oder sequenzielles Trennen der Phasen des Mehrphasensystems in
- eine im Wesentlichen sterolhaltige Phase;
- eine im Wesentlichen glycerin- und methanolhaltige wässrige Phase; und
- eine tocopherolhaltige Methylesterphase;
- ein Gewinnen von Phytosterolen aus der sterolhaltigen Phase; und/oder
- gegebenenfalls ein Gewinnen von Tocopherolen aus der tocopherolhaltige Methylesterphase.

6. Verfahren nach einem der vorhergehenden Ansprüche, **dadurch gekennzeichnet, dass**
die erste Umesterungsstufe mit einem Gehalt an Katalysator im Bereich von 0,1 % bis 0,3 %, bevorzugt im Bereich von 0,18 % bis 0,22 % sowie mit einem Gehalt an Methanol im Bereich von 12 % bis 18 %, bevorzugt im Bereich von 14 % bis 16 % und die zweite Umesterungsstufe mit einem Gehalt an Katalysator im Bereich von 0,5 % bis 1 %, bevorzugt im Bereich von 0,6 % bis 0,8 % sowie mit einem Gehalt an Methanol im Bereich von 30 % bis 38 %, bevorzugt im Bereich von 34 % bis 36 %, jeweils bezogen auf die Masse eines Gesamtansatzes, durchgeführt wird, wobei als Katalysator ein basischer Katalysator, beispielsweise Natriummethylat (Na-Methylat), Natriumhydroxid (NaOH) oder Kaliumhydroxid (KOH) verwendet wird.

7. Verfahren nach einem der vorhergehenden Ansprüche, **dadurch gekennzeichnet, dass**
beim Zugeben von Wasser dieses mit einer Menge im Bereich von 15 % bis 25 %, vorzugsweise im Bereich von 18 % bis 22 % und besonders bevorzugt im Bereich von 19,5 % bis 20,5 %, jeweilis bezogen auf die Masse eines Gesamtansatzes, zugegeben wird, um insbesondere ein Massenverhältnis von Sterolestern : Fettsäuremethylestern : Methanol : Wasser von im Wesentlichen 1 : 2,5 - 3 : 2,2 - 2,5 : 0,8 - 1,2 einzustellen.

8. Verfahren nach einem der vorhergehenden Ansprüche, **dadurch gekennzeichnet, dass**
im Zuge der ersten Umesterungsstufe nach einem Zugeben von Umesterungskomponenten Glycerin in einer Menge im Bereich von 0,2 % bis 7,2 %, bevorzugt im Bereich von 0,5 % bis 6 % und besonders bevorzugt im Bereich von 1 % bis 5,5 %, jeweils bezogen auf die Masse des Gesamtansatzes, zugegeben wird.

9. Verfahren nach einem der vorhergehenden Ansprüche, **dadurch gekennzeichnet, dass**
das Reaktionsgemisch, insbesondere nach dem Zugeben von Wasser durch Mischen, insbesondere Rühren, zu einer Emulsion/Suspension homogenisiert wird.

10. Verfahren nach Anspruch 9, **dadurch gekennzeichnet, dass**
die Emulsion/Suspension auf eine Temperatur unterhalb einer Umesterungstemperatur, insbesondere auf eine Temperatur im Bereich von 5 °C bis 35 °C, vorzugsweise im Bereich von 10, °C bis 30 °C und besonders bevorzugt im Bereich von 15 °C bis 25 °C, abgekühlt wird.

11. Verfahren nach einem der vorhergehenden Ansprüche 9 oder 10,
**dadurch gekennzeichnet, dass**
die Emulsion/Suspension während einer Reifezeit mit einer Dauer, insbesondere im Bereich von 1 Stunde bis 48 Stunden, bevorzugt im Bereich von 2 Stunden bis 36 Stunden und besonders bevorzugt im Bereich von 4 Stunden bis 12 Stunden, gereift wird.

12. Verfahren nach einem der vorhergehenden Ansprüche, **dadurch gekennzeichnet, dass**
das Trennen der Phasen mittels einer Filter-, Sieb und/oder Dekatierzentrifuge durchgeführt wird.

13. Verfahren nach einem der vorhergehenden Ansprüche, **dadurch gekennzeichnet, dass**
die sterolhaltige Phase im Wesentlichen Sterolkristalle aufweist, die mit Methanol in einer Menge im Bereich von 50 % bis 800 %, vorzugsweise im Bereich von 125 % bis 700 % und besonders bevorzugt im Bereich von 200 % bis 550 %, jeweils bezogen auf die Masse der Sterolkristalllphase, gewaschen werden, wobei dieser Methanolwäsche optional eine Verdrängungswäsche der Sterolkristalle mit Methylester, insbesondere Pflanzenölmethylester, wie beispielsweise Raps und/oder Soja und/oder Sonnenblumen und/oder Kokos und/oder Palm und/oder Baumwolilsaatöl und/oder Maiskeimölmethylester, mit einem Mengenverhältnis im Bereich von 50 % bis 500 %, vorzugsweise im Bereich von 75 % bis 400 % und besonders bevorzugt im Bereich von 100 % bis 350 %, jeweils bezogen auf die Masse der Sterolkristallphase, vorausgeht.

14. Verfahren nach Anspruch 13, **dadurch gekennzeichnet, dass**
die Sterollkristalle unmittelbar nach der Methancolwäsche getrocknet und im Anschluss daran, insbesondere ohne weitere Behandlung, beispielsweise Reinigung, verpackt werden.

15. Verfahren nach einem der vorhergehenden Ansprüche, **dadurch gekennzeichnet, dass**
die im Wesentlichen glycerin und methanolhaltige Phase einer Methanolrückgewinnung und/oder das Waschmethanol direkt einer Biodieselanlage zugeführt wird.

16. Verfahren nach einem der vorhergehenden Ansprüche, **dadurch gekennzeichnet, dass**
zu dem Destillationsrückstand vor der ersten und/oder der zweiten Umesterungsstufe Methylester, nämlich Fettsäuremethylester, zugegeben wird, der vorzugsweise durch Destillation aus der tocopherolhaltigen Methylesterphase, abgetrennt wird.

## Claims

1. A method for obtaining phytosterols and/or tocopherols from residues of a distillation of esters of vegetable oils,
**characterized by**
a two-stage alkaline transesterification with an intermediate separation of the glycerin phase, wherein
- in a first step of alkaline transesterification, a transformation of partial glycerides contained in the distillation residues is performed;
- from the reaction mixture immediately resulting from the first step of alkaline transesterification, the glycerin phase is separated; and
- in a second step of alkaline transesterification, a transformation of sterol esters contained in the reaction mixture is performed,
wherein the method is carried out from a two-stage alkaline catalyzed transesterification of a fatty acid methyl ester distillation residue from the biodiesel production with an intermediate separation of a glycerol phase produced in the transesterification for completion of the glyceride reaction in the second reaction stage without removing methanol or catalyst by flashing, distillation or washing.

2. The method according to claim 1,
further **characterized by**
- adding water to the reaction mixture after the second transesterification step for creating a multiphase system;
- simultaneously or sequentially separating the phases of the multiphase system into
- a phase substantially containing sterol;
- an aqueous phase substantially containing glycerin and methanol; and
- a methyl ester phase containing tocopherol;
- obtaining phytosterols from the sterol-containing phase; and/or
- if need be, obtaining tocopherols from the tocopherol-containing methyl ester phase.

3. The method according to any one of the preceding claims,
**characterized in that**
the first and/or the second transesterification step(s) is (are) performed at a temperature in the range from room temperature (25°C) to 88°C, preferably in the range from 40°C to 75°C, and particularly preferred in the range from 55°C to 70°C, and furthermore in particular at normal pressure or atmospheric pressure.

4. A method for obtaining phytosterols and/or tocopherols from residues of a distillation of esters of vegetable oils, preferably from distillation residues from a transesterification of vegetable oils, in particular from the vegetable oil-based fatty acid methyl ester (FAME) production for the biodiesel field of application, **characterized by**
a two-stage alkaline transesterification with an intermediate separation of the glycerin phase, wherein
- in a first step of alkaline transesterification, a transformation of partial glycerides contained in the distillation residues is performed;
- from the reaction mixture immediately resulting from the first step of alkaline transesterification, the glycerin phase is separated; and
- in a second step of alkaline transesterification, a transformation of sterol esters contained in the reaction mixture is performed,
and wherein the first and/or the second transesterification step(s) is (are) performed at a temperature in the range from room temperature (25°C) to 88°C, preferably in the range from 40°C to 75°C, and particularly preferred in the range from 55°C to 70°C, and furthermore in particular at normal pressure or atmospheric pressure.

5. The method according to claim 4,
further **characterized in that**
- adding water to the reaction mixture after the second transesterification step for creating a multiphase system;
- simultaneously or sequentially separating the phases of the multiphase system into
- a phase substantially containing sterol;
- an aqueous phase substantially containing glycerin and methanol; and
- a methyl ester phase containing tocopherol;
- obtaining phytosterols from the sterol-containing phase; and/or
- if need be, obtaining tocopherols from the tocopherol-containing methyl ester phase.

6. The method according to any one of the preceding claims,
**characterized in that**
the first transesterification step is performed at a content of catalyst in the range from 0.1% to 0.3%, preferably in the range from 0.18% to 0.22%, as well as at a content of methanol in the range from 12% to 18%, preferably in the range from 14% to 16%, and the second transesterification step is performed at a content of catalyst in the range from 0.5% to 1%, preferably in the range from 0.6% to 0,8%, as well as a content of methanol in the range from 30% to 38%, preferably in the range from 34% to 36%, in each case relative to the mass of the total batch, wherein an alkaline catalyst, for example, sodium methylate (Na methylate), sodium hydroxide (NaOH) or potassium hydroxide (KOH) is used as a catalyst.

7. The method according to any one of the preceding claims,
**characterized in that**,
when water is added, it is added in an amount in the range from 15% to 25%, preferably in the range from 18% to 22%, and particularly preferred in the range from 19.5% to 20.5%, in each case relative to the mass of the total batch, in particular to adjust a mass ratio of sterol esters : fatty acid methyl esters : methanol : water of substantially 1 : 2.5 - 3 : 2.2 - 2.5 : 0.8 - 1.2.

8. The method according to any one of the preceding claims,
**characterized in that**
in the course of the first transesterification step, after adding transesterification components, glycerin is added in an amount in the range from 0.2% to 7.2%, preferably in the range from 0.5% to 6%, and particularly preferred in the range from 1% to 5.5%, in each case relative to the mass of the total batch.

9. The method according to any one of the preceding claims,
**characterized in that**
the reaction mixture is homogenized to an emulsion/suspension, in particular after adding water, by mixing, in particular stirring.

10. The method according to claim 9,
**characterized in that**
the emulsion/suspension is cooled to a temperature of below a transesterification temperature, in particular to a temperature in the range from 5°C to 35°C, preferably in the range from 10°C to 30°C, and particularly preferred in the range from 15°C to 25°C.

11. The method according to any one of the preceding claims 9 or 10,
**characterized in that**
the emulsion/suspension is matured during a maturation time having a duration in particular in the range from 1 hour to 48 hours, preferably in the range from 2 hours to 36 hours, and particularly preferred in the range from 4 hours to 12 hours.

12. The method according to any one of the preceding claims,
**characterized in that**
the separating of the phases is performed by means of a filter centrifuge, screen centrifuge and/or decanter centrifuge.

13. The method according to any one of the preceding claims,
**characterized in that**
the sterol-containing phase substantially comprises sterol crystals which are washed with methanol in an amount in the range from 50% to 800%, preferably in the range from 125% to 700%, and particularly preferred in the range from 200% to 550%, in each case relative to the mass of the sterol crystal phase, wherein this methanol washing is optionally preceded by a displacement washing of the sterol crystals with methyl ester, in particular vegetable oil methyl ester such as, for instance, methyl ester of rapeseed oil and/or soya oil and/or sunflower oil and/or coconut oil and/or palm oil and/or cottonseed oil and/or corn germ oil, at a proportion in the range from 50% to 500%, preferably in the range from 75% to 400%, and particularly preferred in the range from 100% to 350%, in each case relative to the mass of the sterol crystal phase.

14. The method according to claim 13,
**characterized in that**
the sterol crystals are dried immediately after the methanol washing, and following this, are packaged, in particular without any further treatment, for instance, cleaning.

15. The method according to any one of the preceding claims,
**characterized in that**
the phase substantially containing glycerin and methanol is transferred to a methanol recovery and/or the washing methanol is directly transferred to a biodiesel facility.

16. The method according to any one of the preceding claims,
**characterized in that**
to the distillation residue, prior to the first and/or the second transesterification step(s), a methyl ester, i.e. fatty acid methyl ester is actually added which is preferably separated from the tocopherol-containing methyl ester phase by distillation.

## Revendications

1. Procédé d'obtention de phytostérols et/ou de tocophérols à partir de résidus d'une distillation d'esters d'huiles végétales,
**caractérisé par**
une transestérification basique en deux étapes avec une séparation de la phase glycérine intercalée,
dans lequel
- une réaction des glycérides partiels contenus dans les résidus de distillation est réalisée dans une première étape de transestérification basique ;
- la phase glycérine est séparée à partir d'un mélange de réaction résultant immédiatement de la première étape de transestérification basique ;
et
- une réaction des esters de stérols contenus dans le mélange de réaction est réalisée dans une deuxième étape de transestérification basique,
le procédé étant réalisé à partir d'une transestérification catalysée par une base en deux étapes d'un résidu de distillation d'ester méthylique d'acide gras provenant d'une production de biodiesel avec une séparation intermédiaire d'une phase glycérine résultant de la transestérification pour l'achèvement de la réaction des glycérides au cours de la deuxième étape de réaction sans que le méthanol ou le catalyseur ne soit éliminé par flashage, distillation ou un lavage.

2. Procédé selon la revendication 1, **caractérisé en outre par**
- une addition d'eau au mélange de réaction après la deuxième étape de transestérification pour la production d'un système multiphases ;
- une séparation simultanée ou séquentielle des phases du système multiphases en
- une phase contenant principalement du stérol ;
- une phase aqueuse contenant principalement de la glycérine et du méthanol ; et
- une phase d'ester méthylique contenant du tocophérol ;
- une obtention de phytostérols à partir de la phase contenant du stérol ; et/ou
- le cas échéant une obtention de tocophérols à partir de la phase d'ester méthylique contenant du tocophérol.

3. Procédé selon l'une des revendications précédentes, **caractérisé en ce que**
la première et/ou la deuxième étape de transestérification est réalisée à une température dans la plage de la température ambiante (25 °C) à 88 °C, de préférence dans la plage de 40 °C à 75 °C et particulièrement préférentiellement dans la plage de 55 °C à 70 °C, ainsi qu'en outre en particulier à pression normale et/ou atmosphérique.

4. Procédé d'obtention de phytostérols et/ou de tocophérols à partir de résidus d'une distillation d'esters d'huiles végétales, de préférence de résidus de distillation d'une transestérification d'huiles végétales, en particulier provenant de la production d'ester méthylique d'acide gras à base d'huiles végétales pour le domaine d'application du biodiesel (FAME),
**caractérisé par**
une transestérification basique en deux étapes avec une séparation de la phase glycérine intercalée,
dans lequel
- une réaction des glycérides partiels contenus dans les résidus de distillation est réalisée dans une première étape de transestérification basique ;
- la phase glycérine est séparée à partir d'un mélange de réaction résultant immédiatement de la première étape de transestérification basique ;
et
- une réaction des esters de stérols contenus dans le mélange de réaction est réalisée dans une deuxième étape de transestérification basique,
et la première et/ou la deuxième étape de transestérification est réalisée à une température dans la plage de la température ambiante (25 °C) à 88 °C, de préférence dans la plage de 40 °C à 75 °C et particulièrement préférentiellement dans la plage de 55 °C à 70 °C, ainsi qu'en outre en particulier à pression normale et/ou atmosphérique.

5. Procédé selon la revendication 4, **caractérisé en outre par**
- une addition d'eau au mélange de réaction après la deuxième étape de transestérification pour la production d'un système multiphases ;
- une séparation simultanée ou séquentielle des phases du système multiphases en
- une phase contenant principalement du stérol ;
- une phase aqueuse contenant principalement de la glycérine et du méthanol ; et
- une phase d'ester méthylique contenant du tocophérol ;
- une obtention de phytostérols à partir de la phase contenant du stérol ; et/ou
- le cas échéant une obtention de tocophérols à partir de la phase d'ester méthylique contenant du tocophérol.

6. Procédé selon l'une des revendications précédentes, **caractérisé en ce que**
la première étape de transestérification est réalisée avec une teneur en catalyseur dans la plage allant de 0,1 % à 0,3 %, de préférence dans la plage allant de 0,18 % à 0,22 % ainsi qu'avec une teneur en méthanol dans la plage allant de 12 % à 18 %, de préférence dans la plage allant de 14 % à 16 % et la deuxième étape de transestérification avec une teneur en catalyseur dans la plage allant de 0,5 % à 1 %, de préférence dans la plage allant de 0,6 % à 0,8 % ainsi qu'avec une teneur en méthanol allant dans la plage de 30 % à 38 %, de préférence dans la plage allant de 34 % à 36 %, respectivement par rapport à la masse d'une préparation totale, un catalyseur basique, par exemple le méthylate de sodium (méthylate Na), l'hydroxyde de sodium (NaOH) ou l'hydroxyde de potassium (KOH), étant utilisé comme catalyseur.

7. Procédé selon l'une des revendications précédentes **caractérisé en ce que**
lors de l'addition d'eau celle-ci est additionnée en une quantité dans la plage de 15 % à 25 %, de préférence dans la plage de 18 % à 22 % et particulièrement préférentiellement dans la plage de 19,5 % à 20,5 %, respectivement par rapport à la masse d'une préparation totale, pour régler en particulier un rapport massique des esters de stérols : esters méthyliques d'acide gras/méthanol/eau de sensiblement 1/2,5-3/2,2-2,5/0,8-1,2.

8. Procédé selon l'une des revendications précédentes **caractérisé en ce que**
au cours de la première étape de transestérification après une addition de composants de transestérification la glycérine est ajoutée en une quantité dans la plage allant de 0,2 % à 7,2 %, de préférence dans la plage de 0,5 % à 6 % et particulièrement préférentiellement dans la plage de 1 % à 5,5 %, respectivement par rapport à la masse de la préparation totale.

9. Procédé selon l'une des revendications précédentes, **caractérisé en ce que**
le mélange de réaction, en particulier après l'addition d'eau par mélange, en particulier agitation, est homogénéisé en une émulsion/suspension.

10. Procédé selon la revendication 9, **caractérisé en ce que**
l'émulsion/la suspension est refroidie à une température située en dessous d'une température de transestérification, en particulier à une température dans la plage de 5 °C à 35 °C, en particulier dans la plage allant de 10 °C à 30 °C et particulièrement préférentiellement dans la plage allant de 15 °C à 25 °C.

11. Procédé selon l'une des revendications précédentes 9 ou 10, **caractérisé en ce que**
l'émulsion/la suspension est maturée pendant un temps de maturation ayant une durée, en particulier dans la plage allant de 1 heure à 48 heures, de préférence allant dans la plage de 2 heures à 36 heures et particulièrement préférentiellement dans la plage de 4 heures à 12 heures.

12. Procédé selon l'une des revendications précédentes, **caractérisé en ce que**
la séparation des phases est réalisée au moyen d'une centrifugeuse à filtres, à tamis et/ou par décantation.

13. Procédé selon l'une des revendications précédentes, **caractérisé en ce que**
la phase contenant du stérol présente principalement des cristaux de stérols qui sont lavés avec du méthanol en une quantité allant dans la plage de 50 % à 800 %, de préférence dans la plage de 125 % à 700 %, et particulièrement préférentiellement dans la plage allant de 200 % à 550 %, respectivement par rapport à la masse de la phase de cristaux de stérols, ce lavage au méthanol étant éventuellement précédé d'un lavage par remplacement des cristaux de stérols avec de l'ester méthylique, en particulier de l'ester méthylique d'huiles végétales, comme par exemple de colza et/ou de soja et/ou de tournesol et/ou de coco et/ou de palme et/ou d'huile de graines de coton et/ou d'ester méthylique d'huile de germes de maïs, avec un rapport quantitatif dans la plage de 50 % à 500 %, de préférence dans la plage de 75 % à 400 % et particulièrement préférentiellement dans la plage de 100 % à 350 %, respectivement par rapport à la masse de la phase de cristaux de stérols.

14. Procédé selon la revendication 13, **caractérisé en ce que**
les cristaux de stérols sont séchés directement après le lavage au méthanol et conditionnés consécutivement à cela, en particulier sans autre traitement, par exemple de purification.

15. Procédé selon l'une des revendications précédentes, **caractérisé en ce que**
la phase contenant principalement de la glycérine et du méthanol est renvoyée à une récupération du méthanol et/ou le méthanol de lavage est renvoyé directement à une installation de biodiesel.

16. Procédé selon l'une des revendications précédentes, **caractérisé en ce que**
de l'ester méthylique, notamment de l'ester méthylique d'acide gras, est ajouté au résidu de distillation avant la première et/ou la deuxième étape de transestérification, lequel est de préférence séparé par distillation à partir de la phase d'ester méthylique contenant du tocophérol.
